# EUROPEAN PATENT SPECIFICATION

(11) **EP 2 197 362 B1**
(45) Date of publication and mention of the grant of the patent: **06.01.2016**
(21) Application number: 08798337.5
(22) Date of filing: 21.08.2008
(51) Int. Cl.: A61B 17/04

(54) **BONE ANCHOR COMPRISING A SHAPE MEMORY ELEMENT**
KNOCHENANKER MIT EINEM FORMGEDÄCHTNIS-ELEMENT
ANCRAGE OSSEUX COMPRENANT UN ÉLÉMENT À MÉMOIRE DE FORME

(30) Priority: 24.08.2007 US 966087 P
(43) Date of publication of application: 23.06.2010
(73) Proprietor: C2M Medical, Inc., Edina, MN 55435 (US)
(72) Inventor: KRUMME, John, Woodside, California 94062 (US); MCDEVITT, Dennis, Raleigh, North Carolina 27617 (US)
(74) Representative: Grand, Guillaume
(86) International application number: PCT/US2008/073807
(87) International publication number: WO 2009/029468

(56) References cited:
- EP-A- 0 673 624
- WO-A-00/35355
- WO-A-01/54586
- WO-A-97/30649
- WO-A-98/10693
- FR-A- 2 731 610
- FR-A- 2 736 257
- US-A1- 2003 078 465
- US-A1- 2006 106 422
- US-A1- 2006 282 081

## Description

This present invention relates to apparatus for attaching soft tissue to bone.

Numerous devices are currently available to attach objects to bone. More particularly, screws, staples, cement and sutures have all been used to attach soft tissue (e.g., ligaments, tendons, muscles, etc.) to bone, bone to bone, and inanimate objects (e.g., prostheses) to bone.

Among other things, it can be desirable to anchor a length of conventional suture in bone, so that the free end(s) of the suture can be used to attach a desired object (e.g., a ligament, a tendon, a prostheses, etc.) to the bone. This is typically done by providing a bone anchor having a length of suture attached thereto, and securing the bone anchor in the bone so that the free end(s) of the suture extend from the bone. The free end(s) of the suture can then be used to fasten the desired object to the bone. In practice, such bone anchors have found widespread application in procedures for attaching or re-attaching soft tissue to bone, e.g., to repair a torn rotator cuff in the shoulder.

A wide variety of bone anchors, operating on a number of different principles, have been produced. The commercially-successful bone anchors generally fall into one of four broad categories: (i) "screw-type" bone anchors, (ii) "barb-type" bone anchors, (iii) "expandable casing-type" bone anchors, and (iv) "toggle-type" bone anchors. More particularly, screw-type bone anchors (see, for example, U.S. Patent No. 4,632,100 issued to Goble et al.) generally comprise a screw body which is deployed in the bone by screwing the bone anchor into the bone.

Barb-type bone anchors (see, for example, U.S. Patent No. 4,898,156 to Gatturna et al.) generally comprise a cylindrical body having flexible barbs extending outboard thereof, with the body being deployed in a pre-formed hole in the bone and the barbs engaging the side wall of the bone hole.

Expandable casing-type bone anchors (see, for example, U.S. Patent No. 5,268,001 to Nicholson et al.) generally comprise an expandable body which is deployed in a pre-formed hole in the bone and then the body is expanded so as to engage the side wall of the bone hole.

Toggle-type bone anchors (see, for example, U.S. Patent No. 5,626,612 to Bartlett) generally comprise an elongated body which is inserted in a pre-formed hole in the bone and then the elongated body is turned so as to engage the side wall of the bone hole, whereby to prevent withdrawal from the bone.

It will be appreciated that barb-type bone anchors and expandable casing-type bone anchors share a common feature, i.e., both types of bone anchors enter the bone hole having a first cross-sectional configuration, and thereafter assume a second, larger cross-sectional configuration, whereby to engage the side wall of the bone hole and thereby prevent withdrawal from the bone.

Some bone anchors use barbs formed out of an elastic material which permits them to be elastically deformed inwardly during insertion so as to permit the bone anchor to enter the bone hole. In most cases, a superelastic material is used to form the barbs so as to provide the degree of elasticity required for the application. More particularly, a shape memory alloy (SMA) appropriately engineered to provide monothermal stress-induced martensite (SIM) (e.g., Nitinol) is used. These applications of Nitinol technology for bone anchors are limited to isothermal applications of the stress-induced martensitic (SIM) properties of these shape memory alloys.

In some situations the barbs are sheathed in a tube prior to insertion into the bone hole so as to assume a streamlined geometry. More particularly, in this arrangement, the bone anchor is inserted into the bone hole with its barbs sheathed and, once the bone anchor is properly positioned in the bone hole, the barbs are released so that they spring outwardly and engage the side wall of the bone hole, whereby to anchor the bone anchor in the bone. Unfortunately, the elastically deformed barbs commonly exert high forces on the inside of the sheath, with the result that significant friction is created as the sheath is withdrawn, thus requiring substantial effort to release the barbs from within the sheath.

Alternatively, the barbed anchor may be simply pressed into the pre-drilled hole in the bone, without the barbs being pre-sheathed within a tube. In this situation, the barbs are folded inwardly by virtue of their engagement with the rim of bone at the mouth of the bone hole. Unfortunately, this approach requires high insertion forces and introduces the additional possibility of causing significant displacement or damage to the bone due to engagement of the barbs with the bone as the anchor is inserted. Larger bone holes may be used to reduce the insertion forces required to install the bone anchor, but this is at the expense of reducing the holding power of the anchor within the bone due to the larger bone hole size. Furthermore, larger bone holes increase bone trauma, due to the need to remove more bone material.

Similar problems occur with barb-type bone anchors that use mechanical activation to deploy the barbs, and/or with expandable casing-type bone anchors that use mechanical activation to expand the diameter of the bone anchor.

Furthermore, FR-A-2 737 610 discloses a bone anchor provided with a plurality of temperature activated shape memory alloy engagement elements. The preamble of appended claim 1 is based on this document. However, the bone anchor proposed by FR-A-2 737 610 is necessarily introduced in a pre-drilled bone hole.Besides, in order to lock a suture material on this bone anchor, the body of the bone anchor is provided with a slot with an anti-run-back end, which is not handy to be used.

An object of the invention is to propose a bone anchor that is easier to be used.

The present invention is directed to a bone anchor as defined in appended claim 1.

Advantageous features of the bone anchor according to the invention are specified in appended claims 2 to 15.

In particular, the bone anchor according to the invention uses the temperature transition of a shape memory material to expand the anchor within a bone. The illustrated embodiments are generally directed toward barb-type bone anchors, although applications to expandable casing-type bone anchors will be apparent to those skilled in the art in view of the present disclosure. In one embodiment, the bone anchor uses thermal transformation of the metallic crystal state (and hence the stress/strain properties) of shape memory alloy (e.g., Nitinol, NiTi) elements (e.g., engagement elements), whereby to change the configuration of the bone anchor from a retracted configuration to an expanded configuration.

The present bone anchors are typically provided with engagement elements that protrude from the anchor body in the expanded configuration. In one embodiment, the engagement elements are retained inboard during insertion of the bone anchor into the pre-drilled hole, and then are permitted to spring outwardly so as to engage the side walls of the pre-drilled hole. In another embodiment, the present bone anchor is inserted into the bone without the use of a pre-drilled hole.

The illustrated embodiments significantly reduce (or eliminate) the load complications discussed above with respect to prior art elastic engagement elements, including prior art elastic engagement elements formed out of shape memory alloys. In embodiments where the engagement elements are to be held within a sheath, the use of shape memory alloy elements adapted for appropriate temperature transition reduces or substantially eliminates the internal forces exerted by the engagement elements on an overlying sheath, thus resulting in easier loading of the barbed anchor into the sheath and easier (i.e., lower friction) release of the anchor from the sheath.

Correspondingly, where the bone anchor is to be set into the bone without using an overlying sheath, the use of shape memory elements with appropriate temperature transition substantially reduces the insertion forces needed to insert unsheathed engagement elements, thereby facilitating anchor deployment and permitting the use of smaller pre-drilled holes which in turn provides better anchor gripping and reduced bone trauma. Furthermore, the present bone anchors are particularly well suited for self-embedding insertion into bone without the use of pre-drilled holes.

The present invention is also applicable to expandable casing-type bone anchors. Significantly, the ease of insertion of bone anchors formed in accordance with the present invention, and the resulting higher pull-out forces produced by such bone anchors, equal or exceed those of comparable devices not incorporating the present invention.
Fig. 1 illustrates a bone anchor and inserter in accordance with an embodiment which is not according to the present invention.
Fig. 2 illustrates the bone anchor of Fig. 1 in a deployed configuration.
Fig. 3 illustrates the bone anchor of Fig. 1 in a retracted configuration.
Fig. 4 illustrates the bone anchor of Fig. 1 in an expanded configuration.
Fig. 5 is a stress vs. strain graph for a bone anchor.
Fig. 6 is a strain recover vs. temperature graph for a bone anchor.
Figs.7 and 8 illustrate an exemplary inserter with a resistance heater.
Figs. 9 and 10 illustrate an exemplary locking assembly for a bone anchor in accordance with an embodiment which is not according to the present invention.
Fig. 11 illustrates an inserter.
Fig. 12 illustrates a suture loading mechanism.
Figs. 13A and 13B illustrate a bone anchor loaded in the inserter of Fig. 11.
Figs. 14A and 14B illustrate operation of the inserter of Fig. 11.
Figs. 15-17 illustrate an alternate locking assembly for a bone anchor in accordance with an embodiment of the present invention.
Figs. 18-20 illustrate another locking assembly for a bone anchor in accordance with an embodiment of the present invention.
Figs. 21A-24 illustrate an alternate inserter.

The present invention is directed to a bone anchor that includes temperature-activated engagement elements formed from a shape memory metal, such as for example a NiTi material sold by NDC Corporation of Fremont, CA and designated SE 508 (50.8 % Ni content). As used herein, "engagement elements" refers to a temperature activated, shape memory alloy structure adapted to retain a bone anchor in bone. The engagement elements can have a variety of configurations, such as for example, barbs, hooks, serrated or threaded members, and the like.

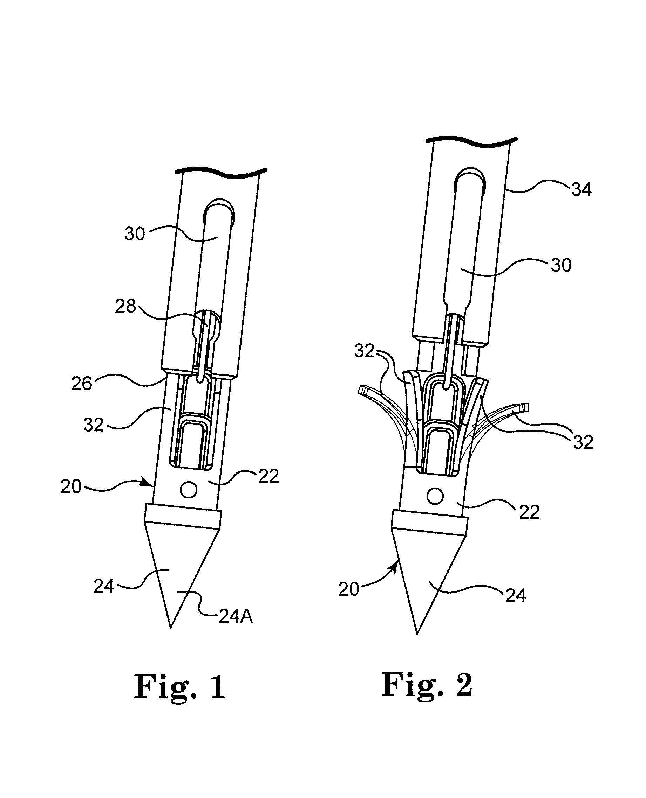

One example of the present bone anchor 20 is shown in Figs. 1 and 2. The bone anchor 20 generally includes a body 22 having a distal end 24 and a proximal portion 26. Retaining structure 28 holds the proximal portion 26 in engagement with inserter 30. In Fig. 1 a plurality of shape memory alloy engagement elements 32 are in a retracted configuration. In Fig. 2 the engagement elements 32 are in a radially expanded configuration relative to the body 22. Retractable sheath 34 preferably overlies the engagement elements 32 and proximal portion 26 of the body 22 for structural support during insertion. Retractable sheath 34 is withdrawn after the bone anchor 20 is positioned in the bone. Furthermore, the bone anchor 20 shown in Figs. 1 and 2 may omit the sheath 34, relying on temperature control to maintain the engagement elements 32 in their proper radial position.

The distal end 24 of the bone anchor 20 is preferably a self-embedding tip 24A that does not require a pre-drilled hole. Alternatively, the tip 24A my include threads, teeth, chiseled cutting edges, tip blades, or other structures that permit the bone anchor 20 to be inserted through cortical bone without a pre-drilled hole.

The shape memory alloy engagement elements 32 are preferably configured so that when in the retracted configuration they normally lie within about the maximum cross-section of the body 22 during anchor insertion. In the illustrated embodiment, the pointed distal end 24 has a cross section greater than, or equal to, the cross section of the engagement elements 32 in the retracted configuration. The engagement elements 32 extend well beyond the cross section of the distal end 24 when in the expanded configuration, as illustrated in Fig. 2.

The engagement elements 32 are temperature-activated once the bone anchor 20 is in place so as to cause them to move to an expanded configuration. In the expanded configuration the engagement elements 32 extend radially outward from the body 22 and press against the bone to fix the bone anchor 20. Thus, the shape memory alloy engagement elements 32 are activated from the retracted configuration to the expanded configuration through temperature transition upon insertion. This is in marked contrast to prior art elastic barb constructions, which are designed to harness the isothermal SIM elastic characteristics of the shape memory alloy.

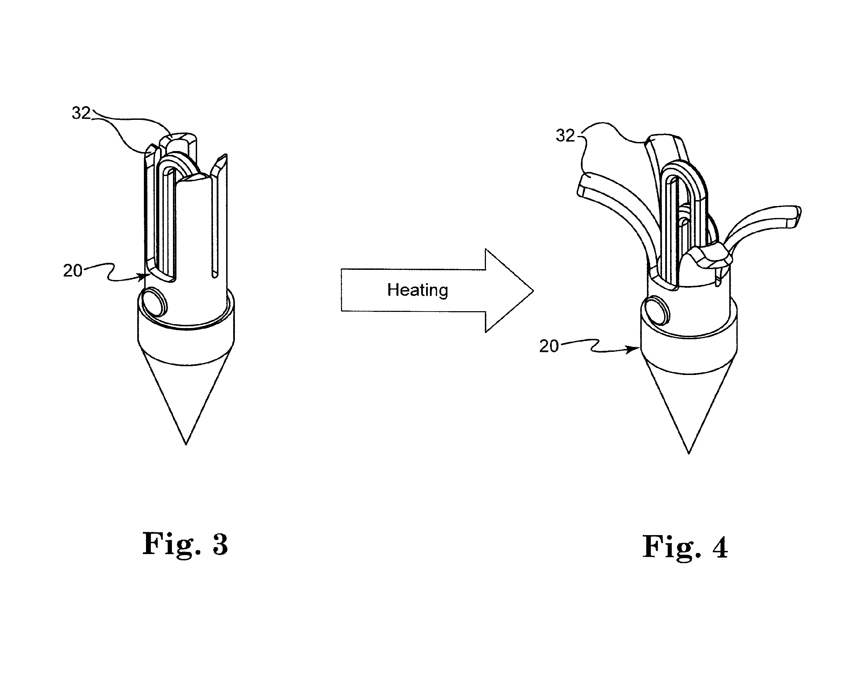

Figs. 3 and 4 illustrate the bone anchor 20 without the inserter 30 for the sake of clarity. The shape memory alloy engagement elements 32 of the illustrated embodiment are preferably formed and heat-treated so as to achieve an As (Austenitic start temperature) of about 25- 27° C and an Af (Austenitic finish temperature) of about 30-35° C. This Af temperature is above that of a normal surgical operating room temperature (which is about 20° C), and just below that of normal body temperature (which is 37° C). Thus, the shape memory alloy engagement elements 32 of the bone anchor 20 will be below their As temperature while at operating room temperature (20° C) and will be at or above their Af temperature when positioned in the patient's body. Transition from the retracted configuration to the expanded configuration typically occurs in a temperature band or range between the As (Austenitic start temperature) and the Af (Austenitic finish temperature).

By constructing the bone anchor 20 so that the shape memory alloy engagement elements 32 have the retracted configuration shown in Fig. 3 while below their As temperature, and have the expanded configuration shown in Fig. 4 while at or above their Af temperature, thermal transition of the shape memory alloy engagement elements 32 (i.e., from As to Af) can be used to deploy the engagement elements 32 after the anchor 20 has been positioned in the bone.

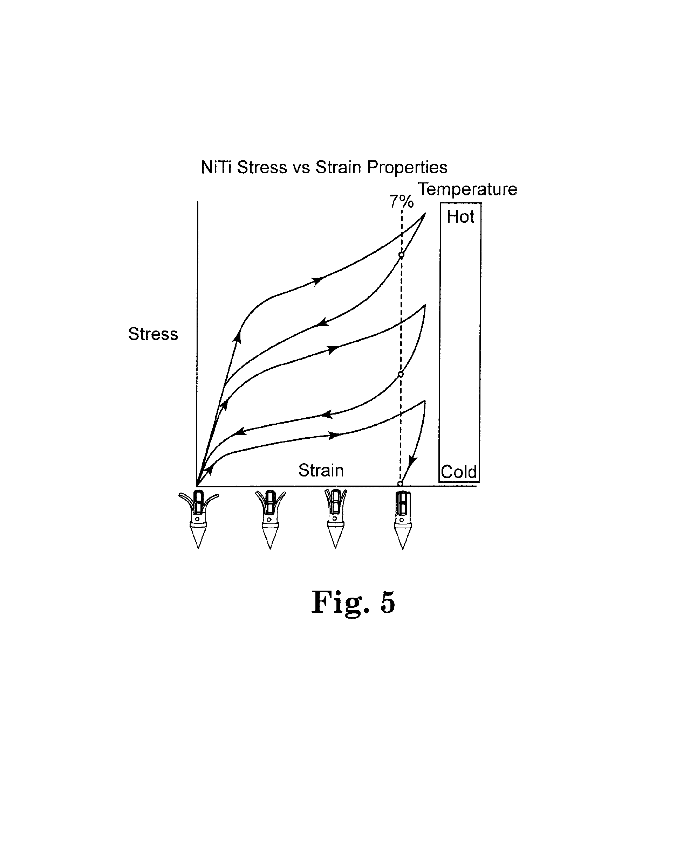

At the ambient conditions in the operating room (about 20° C) the engagement elements 32 of the bone anchor 20 are in a martensitic crystal structure state. In this state, the engagement elements 32 exhibit the stress/strain properties as shown in Fig. 5 (at the "cold" temperature). The bone anchor 20 is then installed in the bone and thus warmed up to the patient's body temperature by the environment in the bone, i.e., at or above its Af temperature. This warming process transforms the engagement elements 32 crystal structure from the martensitic state to the austenitic state, (which exists at Af and above). At this point, the engagement elements 32 exhibit the stress/strain properties as shown in Fig. 5 (at "hot" temperature).

A vertical line in Fig. 5 represents a constant strain condition. The 7% line, for example, represents the strain condition of the engagement elements 32 as the sheath is removed and the engagement elements 32 encounter the surrounding bone. As the engagement elements 32 reach a temperature above As (austenitic start temperature) and then at or above Af (austenitic finish temperature), the stress level rises as shown in the "hot" (top) curve and the engagement elements 32 begin to penetrate the bone. Penetration of the bone corresponds to decreasing strains as demonstrated by the anchor engagement elements 32 moving outward.

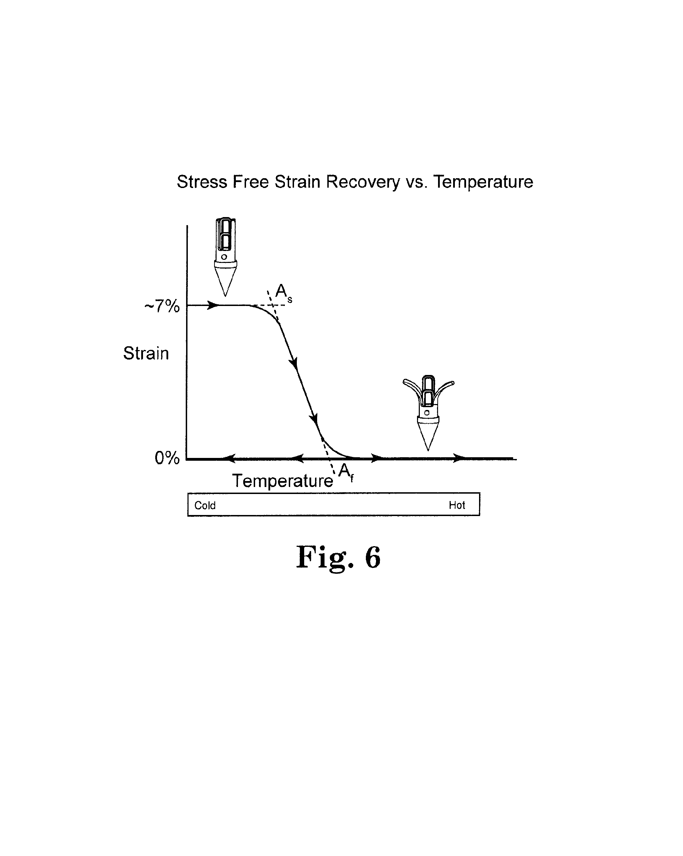

As illustrated in Fig. 6, the engagement elements 32, which are now at a temperature at or above Af, attempt to assume the shape shown in Fig. 4, thereby causing the engagement elements 32 to expand within the bone, thereby fixing the bone anchor 20 in the bone. Fig. 6 defines As (austenitic start temperature) and Af (austenitic finish temperature) as the material crystal structure transforms from the martensitic state to the austenitic state with increasing temperature. The present invention involves processing the anchor engagement elements so that As is about 25° C to about 27° C and Af is about 33° C to about 35° C. The graphic anchor device illustrations in Fig. 6 show the stress-free shape strain recovery of the bone anchor 20 in accordance with the illustrated embodiment. Additional background of implantable medical devices of shape memory alloy is found in U.S. Patent No. 5,876,434 (Flomenblit et al.).

External heating can optionally be applied to the bone anchor 20 to accelerate temperature transition of the shape memory alloy to Af and above. By way of example but not limitation, this can be done by locally applying a warm (e.g., >37°C) saline bath to the bone anchor 20 through a syringe or through the inserter attached to the bone anchor.

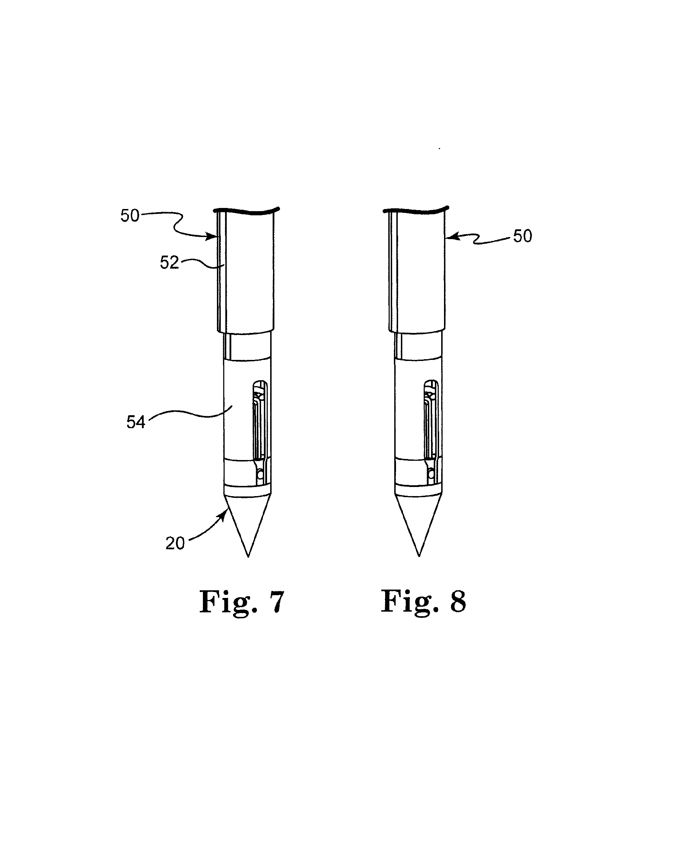

Other methods may be used to speed up warming of the bone anchor 20 to a temperature at or above Af. For example, as illustrated in Figs. 7 and 8, the inserter/delivery device 50 can optionally incorporate electrodes 52 electrically coupled to resistance heater 54. By applying a voltage to the electrodes 52 the resistance heater 54 heats the bone anchor 20 causing the anchor engagement elements 32 to open (see Fig. 4). Other methods of heating the bone anchor 20 locally in position in the bone include, for example, electromagnetic induction, delivery of electromagnetic energy through an optical fiber located in the inserter/delivery device 42, conductive or convective heating, and the like.

The use of a temperature-activated, shape memory alloy is applicable to a wide variety of bone anchor structures, inserters, and suture attachment mechanisms, such as disclosed in commonly assigned U.S. Patent Publication Nos. 20070260259 entitled Bone Anchor Installer And Method Of Use; 20070255317 entitled Suture Passer Devices And Uses Thereof; 20070156176, 20070156150, 20070156149, and 20070156148 all entitled Ring Cinch Assembly To Attach Bone To Tissue; 20060282083, 20060282082 and 20060282081 all entitled ; Apparatus And Method For Securing Tissue To Bone With A Suture; and 20050245932 entitled Apparatus And Methods For Securing Tissue To Bone.

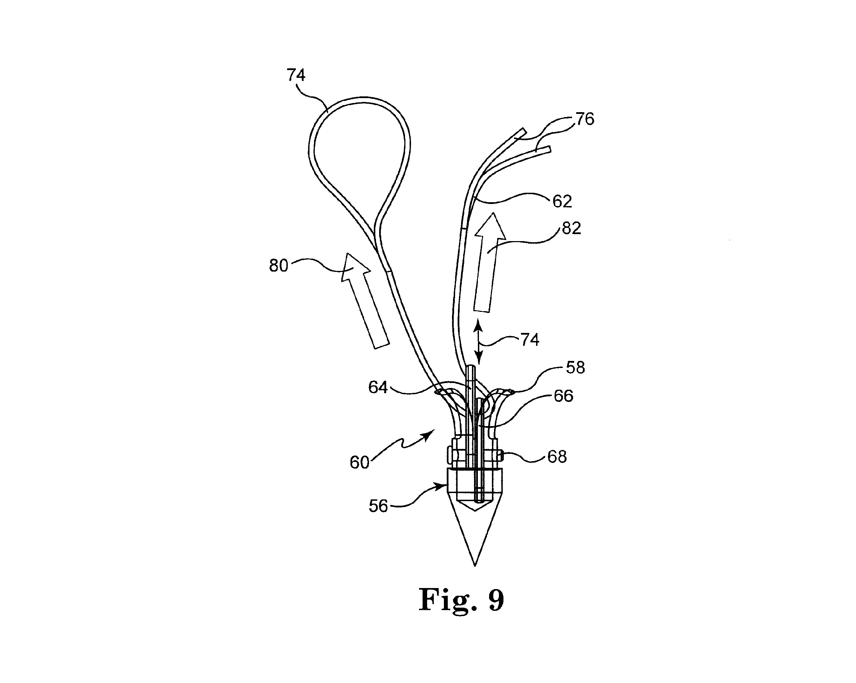

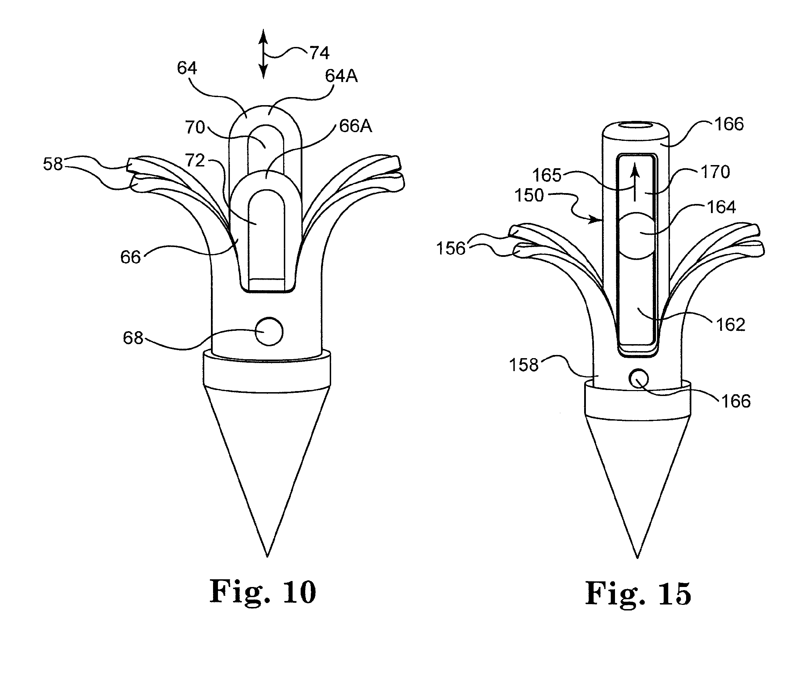

Figs. 9 and 10 illustrate an example of a self-locking assembly 60 for attaching the suture 62 to the bone anchor 56. The operation and structure of the self-locking assembly 60 are preferably independent of the operation and structure of the engagement elements 58.

The bone anchor 20 includes a pair of locking rings 64, 66. Pin 68 extends through openings70, 72 to attach the locking rings 64, 66, respectively. The locking rings 64, 66 are preferably positioned parallel to each other and can move axially relative to each other along axis 74.

As illustrated in Fig. 9, the suture 62 is threaded through the opening 70 of the locking ring 64 from the side opposite the locking ring 66. The suture 62 is then wrapped around upper bar 66A of locking ring 66 and then back through the opening 72.

The self-locking assembly 60 of Fig. 9 permits the suture 62 to be reversibly locked in place. When a tension force 80 is applied to first end 74 of the suture 62, the locking ring 66 is pulled upward into engagement with the locking ring 64 to compressing the suture 62 between upper bars 64A, 66A of the locking rings 64, 66, respectively. Alternatively, when a tension force 82 is applied to the second end 76 of the suture 62, the load is carried primarily by the upper bar 64A of the locking ring 64. The tension force 82 does not pull the upper bars 64A, 66A into engagement, hence, the suture 62 slides freely through the self-locking assembly 60. The self-locking assembly 60 described above is self-locking. As used herein, "self-locking assembly" refers to a mechanism for attaching a suture to a bone anchor that resists a tension force on the suture in at least one direction. The engagement elements 58 are preferably design to expand further, thereby increasing engagement with the bone, if a tension force is applied to the bone anchor 56 in the directions 80 or 82.

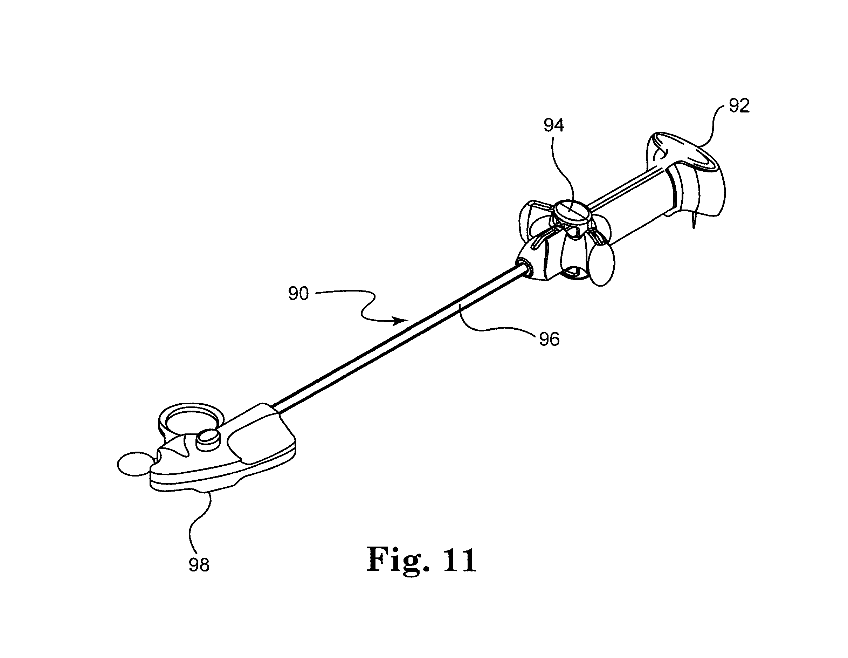

The presently described embodiments may optionally be provided in combination with an installer system adapted for use in deploying the presently described bone anchors to the operative site. Figs. 11-14 illustrate an exemplary anchor installer 90 in accordance with an embodiment of the present invention. The anchor installer 90 includes a proximal handle 92, a deployment lock-out mechanism 94, an elongated shaft 96, and a suture loading mechanism 98.

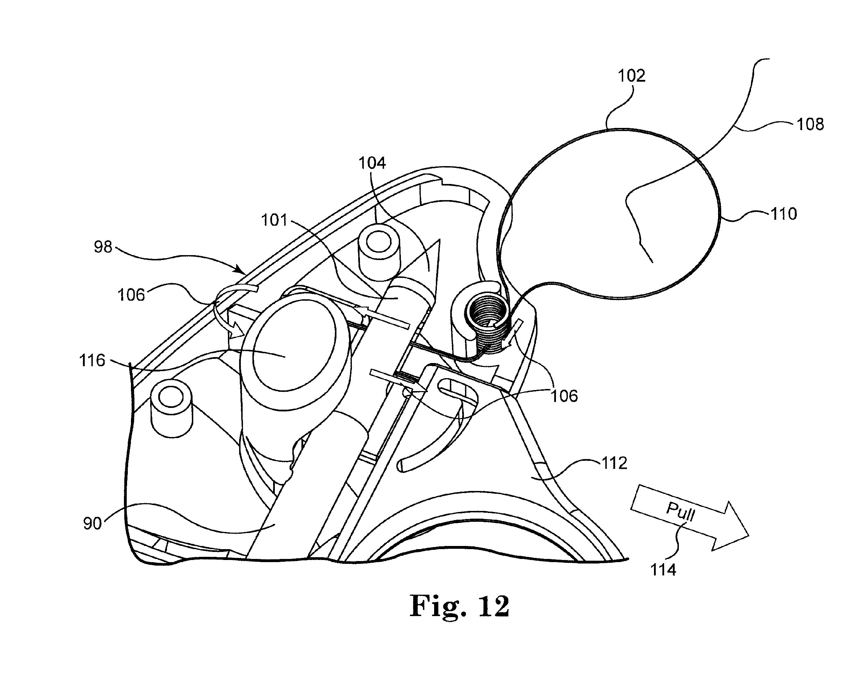

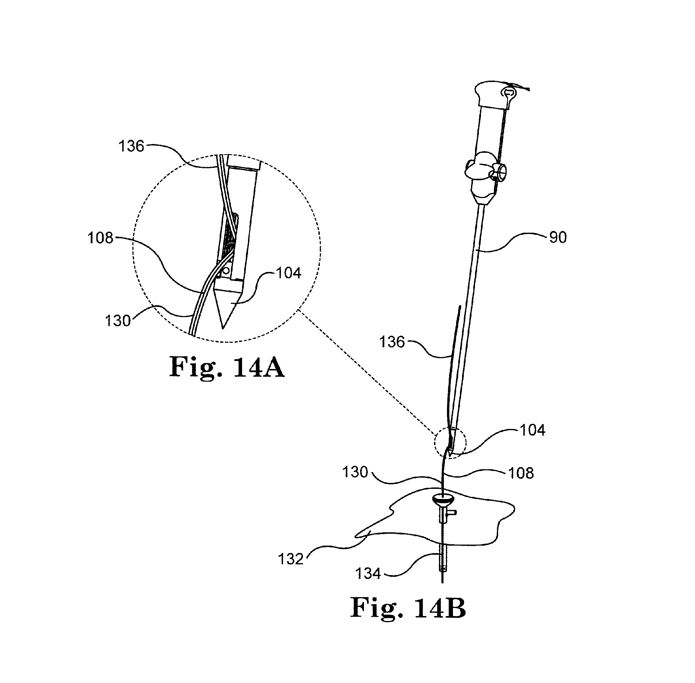

As best illustrated in Fig. 12 the suture loading mechanism 98 holds bone anchor 104 in place at the distal end 101 of the anchor installer 90 prior to the deployment into the bone. Wire 102 is pre-threaded through the bone anchor 104. The path of the wire 102 is identified by arrows 106. To load suture 108 (see Fig. 14) in the bone anchor 104, a segment of suture 108 is positioned in loop 110 of the wire 102. Pull-ring 112 is disengaged from the suture loading mechanism 98 and pulled in the direction 114. This action feeds the suture 108 through the bone anchor 104. Pin or button 116 is then depressed to detach the suture loading mechanism 98 from the anchor installer 90.

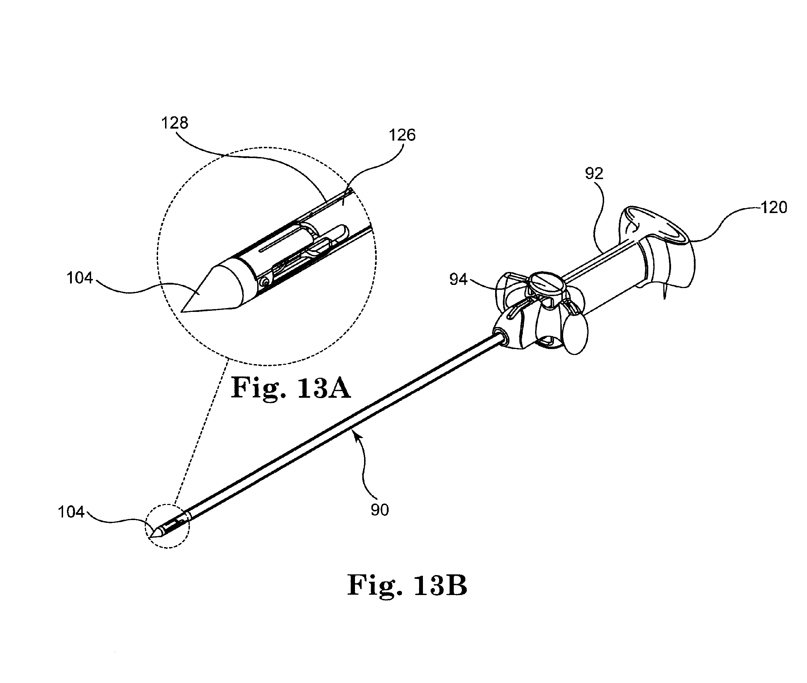

Figs. 13A and 13B illustrate the anchor installer 90 loaded with bone anchor 104. Deployment lock-out mechanism 94 is depressed. As proximal end 120 is advance with respect to the handle 92 an ejector sleeve 126 deploys the bone anchor 104 from outer sleeve 128.

Figs. 14A and 14B illustrate operation of the anchor installer 90. First end 130 of the suture 108 is attached to tissue 132, either directly or using attachment mechanism 134. Various attachment mechanisms 134 are disclosed in commonly assigned U.S. Patent US2008188936 entitled System and Method for Repairing Tendons and Ligaments, filed February 4, 2008. The suture 108 is then loaded in the anchor installer 90 using the suture loading mechanism 98 illustrated in Fig. 12. The bone anchor 104 is then deployed in a desired location in the bone. The second end 136 is then tensioned to pull the tissue 132 in the desired location. See e.g., Fig. 9. Once the tissue 132 is in the desired position, excess portions of the second end 136 of the suture 108 are removed.

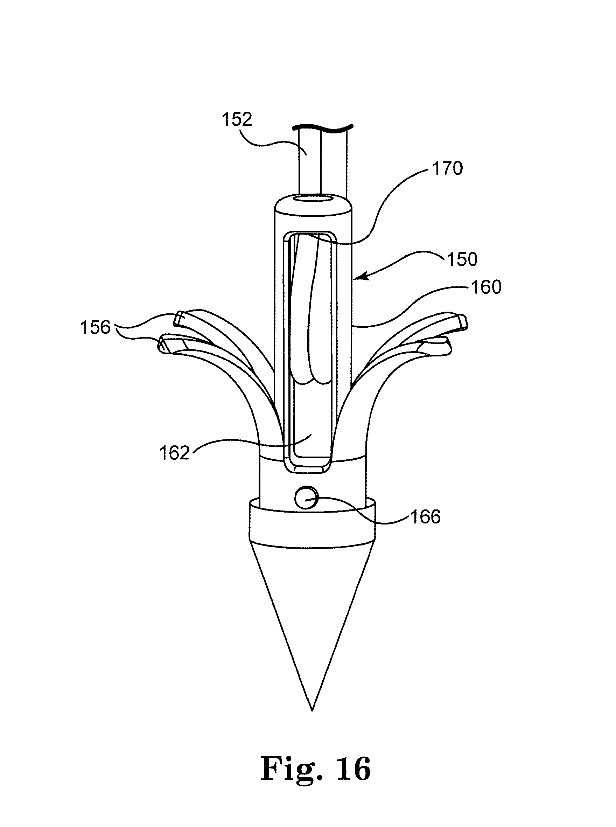

Figs. 15 through 17 illustrate self-locking assembly 150 according to the invention for attaching the suture 152 to the bone anchor 154. The operation and structure of the self-locking assembly 150 are preferably independent of the operation and structure of the engagement elements 156. Proximal end 158 of the bone anchor 154 includes a housing 160 with a slot 162. Ball 164 is located in the slot 162. The housing 160 is preferably attached to the proximal end 158 of the bone anchor 154 by pin 166. The ball 164 is retained in the slot 162, but moves freely along axis 165.

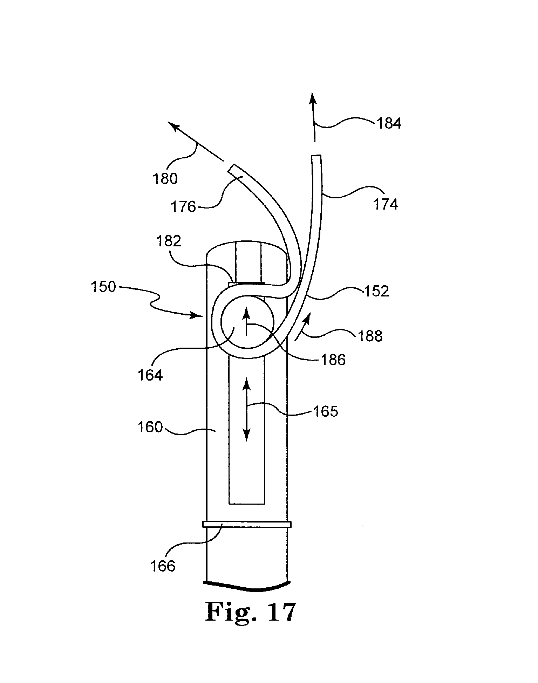

In one embodiment, suture 152 is fed through upper portion 170 of the slot 162 above the ball 164, around the ball 164, and then back out through lower portion 172 of the slot 162 below the ball 164. As best illustrated in Fig. 17, first end 174 of the suture 152 is attached to tissue (See e.g., Fig. 14). The bone anchor 154 is then installed in the desired location of the bone. A tension force 180 is then applied to the second end 176 to pull the tissue to the desired location. The tension force 180 creates opposing tension force 184. This opposing tension force 184 retains the ball 164 at the top 182 of the slot 162, creating compression force 186 that compresses the suture 152 between the ball 164 and the housing 160. The result is a reversible, self-locking locking assembly 150.

The tension force 184 on the tissue can be reduce by pulling on the first end 174 of the suture 152 to reduce the compression force 186 exerted by the ball 164. The surgeon can advance the suture 152 in the reverse direction 188.

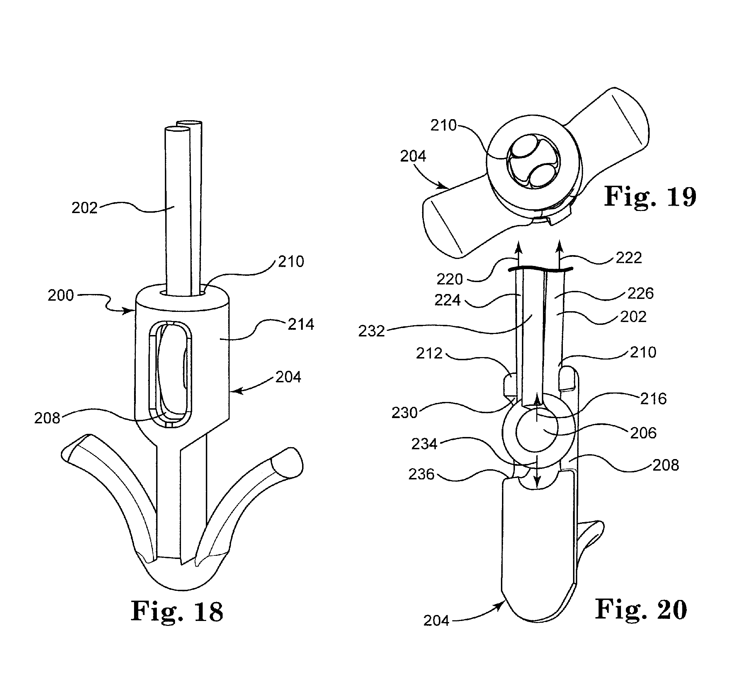

Figs. 18-20 illustrate an alternate self-locking assembly 200 for attaching the suture 202 to the bone anchor 204. The self-locking assembly 200 uses a ball 206 and slot 208 arrangement similar to that illustrated in Figs. 15-17.

As best illustrated in Fig. 20, the suture 202 passes through hole 210 in proximal end 212 of housing 214. The suture 202 is wrapped around the ball 206 and extends back through the hole 210. Tension forces 220, 222 on either end 224, 226 of the suture 202 urges the ball 206 to top 230 of the slot 208, creating a compression force 216 that compresses the suture 202 against the top 230 of the slot 208.

In the illustrated embodiment, rod 232 is provided to move the ball 206 out of engagement with the top 230 of the slot 208, thereby permitting the suture 202 to be moved through the bone anchor 204. During insertion of the bone anchor 204 the rod 232 also pushes the ball 206 downward within the slot 208, creating a compression force 234 that compresses the suture 202 between the ball 206 and bottom 236 of the slot 208.

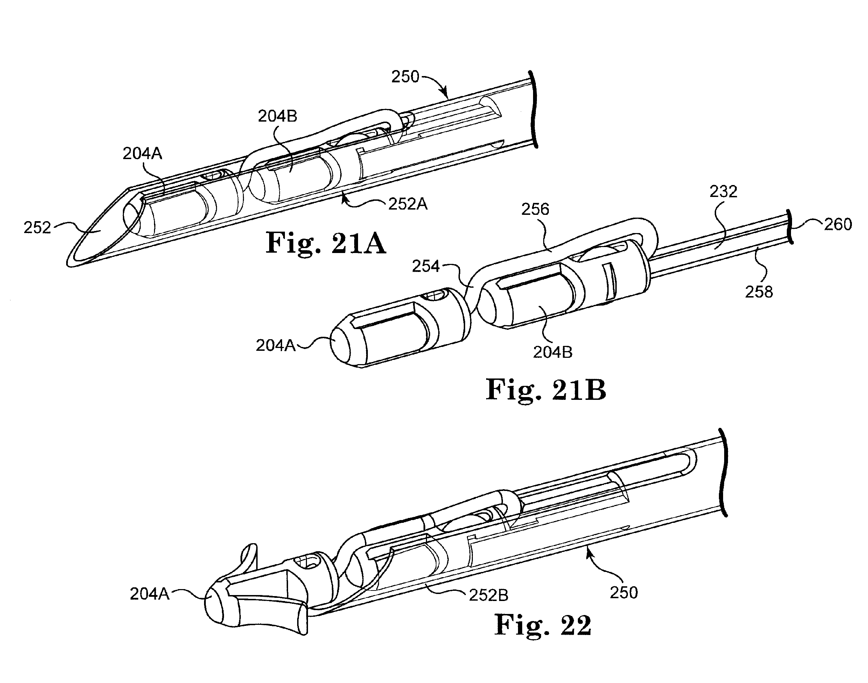

Figs. 21-24 illustrate an inserter 250 for use with the bone anchor 204 illustrated in Figs. 18-20. Fig. 21A illustrates a needle 252 in a first position 252A containing first bone anchor 204A and second bone anchor 204B. Fig. 21B illustrates the bone anchors 204A, 204B with the needle 252 removed for clarity.

Distal end 254 of suture 256 is attached to the first bone anchor 204A using conventional techniques. The suture 256 engages with the second bone anchor 204B generally as illustrated in Figs. 18-20. Rod 232 is provided to release compression force 216 on the suture 256 (see Fig. 20). Proximal end 258 of the suture 256 extends toward proximal end 260 of the rod 232.

Fig. 22 illustrates the first bone anchor 204A ejected from the inserter 250. In the preferred embodiment, the needle 252 is inserted into the bone and the rod 232 retains the bone anchor 204A within the bone while the needle 252 is retracted to a second position 252B.

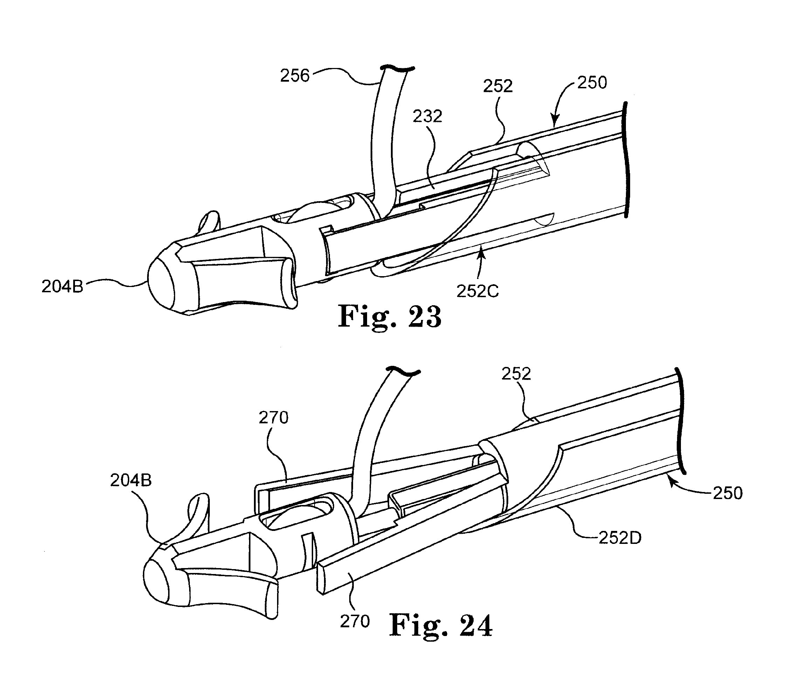

Fig. 23 illustrates the second bone anchor 204B ejected from the inserter 250. In the preferred embodiment, the needle 252 is inserted into the bone and the rod 232 retains the bone anchor 204B in place while the needle 252 is retracted to a third position 252C. In the preferred embodiment, the rod 232 pushes the ball to the bottom of the slot, thereby preventing the suture 256 from moving relative to the bone anchor 204B during insertion. (See e.g., Fig. 20).

Fig. 24 illustrates the needle 252 retracted to a fourth position 252D. Spring legs 270 that previously retained the bone anchor 204B to the inserter 250 are free to expand, thereby releasing the bone anchor 204B. Operation of the bone anchor 204B continues as outlined in Figs. 18-20.

Where a range of values is provided, it is understood that each intervening value, to the tenth of the unit of the lower limit unless the context clearly dictates otherwise, between the upper and lower limit of that range and any other stated or intervening value in that stated range is encompassed within the inventions. The upper and lower limits of these smaller ranges which may independently be included in the smaller ranges is also encompassed within the inventions, subject to any specifically excluded limit in the stated range. Where the stated range includes one or both of the limits, ranges excluding either both of those included limits are also included in the inventions.

Unless defined otherwise, all technical and scientific terms used herein have the same meaning as commonly understood by one of ordinary skill in the art to which these inventions belong. Although any methods and materials similar or equivalent to those described herein can also be used in the practice or testing of the present inventions, the preferred methods and materials are now described. All patents and publications mentioned herein, including those cited in the Background of the application, disclose and describe the methods and/or materials in connection with which the publications are cited.

The publications discussed herein are provided solely for their disclosure prior to the filing date of the present application. Nothing herein is to be construed as an admission that the present inventions are not entitled to antedate such publication by virtue of prior invention. Further, the dates of publication provided may be different from the actual publication dates which may need to be independently confirmed.

Other embodiments of the invention are possible. Although the description above contains many specificities, these should not be construed as limiting the scope of the invention, but as merely providing illustrations of some of the presently preferred embodiments of this invention. It is also contemplated that various combinations or subcombinations of the specific features and aspects of the embodiments may be made and still fall within the scope of the inventions. It should be understood that various features and aspects of the disclosed embodiments can be combined with or substituted for one another in order to form varying modes of the disclosed inventions. Thus, it is intended that the scope of at least some of the present inventions herein disclosed should not be limited by the particular disclosed embodiments described above.

Thus the scope of this invention should be determined by the appended claims. Therefore, it will be appreciated that the scope of the present invention fully encompasses other embodiments which may become obvious to those skilled in the art, and that the scope of the present invention is accordingly to be limited by nothing other than the appended claims, in which reference to an element in the singular is not intended to mean "one and only one" unless explicitly so stated, but rather "one or more." All structural, chemical, and functional equivalents to the elements of the above-described preferred embodiment that are known to those of ordinary skill in the art are intended to be encompassed by the present claims. Moreover, it is not necessary for a device or method to address each and every problem sought to be solved by the present invention, for it to be encompassed by the present claims. Furthermore, no element, component, or method step in the present disclosure is intended to be dedicated to the public regardless of whether the element, component, or method step is explicitly recited in the claims.

## Claims

1. A bone anchor system for coupling tissue to bone, the bone anchor system comprising:
a body portion (154; 204) comprising a proximal end (158) and a distal end adapted to be inserted into the bone;
a tip on the distal end of the body portion;
a self-locking assembly (150; 200) located at the proximal end of the body portion;
suture material (152; 202) engaged with the self-locking assembly; and
a plurality of temperature activated shape memory alloy engagement elements (156) attached to the body portion and maintained in a retracted configuration at a first temperature, and moveable to an expanded configuration at a second temperature greater than the first temperature,
**characterized in that** the tip is adapted to penetrate the bone without a pre-drilled hole and **in that** the locking assembly (150; 200) comprises a ball (164; 206) sliding along a longitudinal axis (165) of the body portion in a slot (162; 208) located at the proximal end (158) of the body portion (154; 204).

2. The bone anchor system of claim 1 wherein the first temperature is about 25°C to about 27° C and the second temperature is about 30°C to about 35° C.

3. The bone anchor system of any one of the preceding claims wherein movement of the engagement elements (156) from the retracted configuration to the expanded configuration occurs in a temperature range between the first temperature and the second temperature.

4. The bone anchor system of any one of the preceding claims wherein the tip comprises a cross section greater than a cross section of the engagement elements (156) in the retracted configuration.

5. The bone anchor system of any one of the preceding claims wherein the engagement elements (156) expand radially outward from the body portion (154; 204) when in the expanded configuration.

6. The bone anchor system of any one of the preceding claims wherein the engagement elements comprise barbed structures.

7. The bone anchor system of any one of the preceding claims wherein the engagement elements (156) are configured to substantially inhibit removal of the bone anchor from the bone.

8. The bone anchor system of any one of the preceding claims wherein the locking assembly (200) comprises:
a housing (214) provided with the slot (208) and located at the proximal end of the body portion (204); and
an opening (210) at a proximal end (212) of the slotted housing (214);
wherein the ball (206) is slidable within the slotted housing (214); and
wherein the suture material (202) is threaded through the opening (210), around the ball (206) and back out through the opening (210).

9. The bone anchor system of claim 8 comprising a rod (232) sized to extend through the opening (210) at the proximal end (212) of the slotted housing (214) to displace the ball (206) within the slot (208).

10. The bone anchor system of any one of claims 8 or 9 wherein tension on the suture material (202) moves the ball (206) to a top (230) of the slot (208) and compresses the suture material between the ball and the top of the slot.

11. The bone anchor system of any one of claims 1 to 7 wherein the locking assembly (150) comprises a housing (160) provided with the slot (162) and located at the proximal end (158) of the body portion (154);
wherein the ball (164) is slidable within the slotted housing (160); and
wherein the suture material (152) is threaded into the slot (162) above the ball (164), around the ball, and back out the same slot below the ball.

12. The bone anchor system of claim 11 wherein a tension force on a first end (174) of the suture material (152) advances the ball (164) to a top (182) of the slot (162), creating a compression force that compresses the suture between the ball and the top of the slot.

13. The bone anchor system of any one of the preceding claims comprising an inserter (50; 90; 250) attached to the proximal end (158) of the body portion (154; 204).

14. The bone anchor system of claim 13 wherein the inserter (50; 90; 250) prevents movement of the engagement elements (156) from the retracted configuration to the expanded configuration.

15. The bone anchor system of any of claims 13 or 14 wherein the inserter (50; 90; 250) comprises a heating mechanism (54) to increase the temperature of the engagement elements (156) to the second temperature.

## Patentansprüche

1. Ein Knochenankersystem zum Kuppeln von Gewebe mit Knochen, das Knochenankersystem aufweisend:
einen Körperabschnitt (154; 204), welcher ein proximales Ende (158) und ein distales Ende, das angepasst ist, um in den Knochen eingesetzt zu sein, aufweist,
eine Spitze am distalen Ende des Körperabschnitts,
eine Selbstverriegelungsbaugruppe (150; 200), welche sich am proximalen Ende des Körperabschnitts befindet,
Nahtmaterial (152; 202), welches mit der Selbstverriegelungsbaugruppe im Eingriff ist, und
eine Mehrzahl von temperaturaktivierten Formgedächtnislegierung-Eingriffselementen (156), welche am Körperabschnitt befestigt ist und bei einer ersten Temperatur in einer Gefaltet-Konfiguration gehalten wird und bei einer zweiten Temperatur, welche größer ist als die erste Temperatur, in eine Entfaltet-Konfiguration bewegbar ist,
**dadurch gekennzeichnet, dass** die Spitze angepasst ist, um ohne ein vorgebohrtes Loch in den Knochen einzudringen, und dass die Verriegelungsbaugruppe (150; 200) eine Kugel (164; 206) aufweist, welche entlang einer Längsachse (165) des Körperabschnitts in einem Schlitz (162; 208), der sich am proximalen Ende (158) des Körperabschnitts (154; 204) befindet, verschiebbar ist.

2. Das Knochenankersystem gemäß Anspruch 1, wobei die erste Temperatur etwa 25°C bis etwa 27°C ist und die zweite Temperatur etwa 30°C bis etwa 35°C ist.

3. Das Knochenankersystem gemäß irgendeinem der vorhergehenden Ansprüche, wobei eine Bewegung der Eingriffselemente (156) ausgehend von der Gefaltet-Konfiguration zur Entfaltet-Konfiguration in einem Temperaturbereich zwischen der ersten Temperatur und der zweiten Temperatur auftritt.

4. Das Knochenankersystem gemäß irgendeinem der vorhergehenden Ansprüche, wobei die Spitze einen Querschnitt aufweist, welcher größer ist als ein Querschnitt der Eingriffselemente (156) in der Gefaltet-Konfiguration.

5. Das Knochenankersystem gemäß irgendeinem der vorhergehenden Ansprüche, wobei die Eingriffselemente (156) ausgehend vom Körperabschnitt (154; 204) radial auswärts entfaltet sind, wenn sie sich in der Entfaltet-Konfiguration befinden.

6. Das Knochenankersystem gemäß irgendeinem der vorhergehenden Ansprüche, wobei die Eingriffselemente Widerhakenstrukturen aufweisen.

7. Das Knochenankersystem gemäß irgendeinem der vorhergehenden Ansprüche, wobei die Eingriffselemente (156) eingerichtet sind, um im Wesentlichen ein Entfernen des Knochenankers vom Knochen zu unterbinden.

8. Das Knochenankersystem gemäß irgendeinem der vorhergehenden Ansprüche, wobei die Verriegelungsbaugruppe (200) aufweist:
ein Gehäuse (214), welches mit dem Schlitz (208) bereitgestellt ist und sich am proximalen Ende des Körperabschnitts (204) befindet, und
eine Öffnung (210) an einem proximalen Ende (212) des geschlitzten Gehäuses (214),
wobei die Kugel (206) innerhalb des geschlitzten Gehäuses (214) verschiebbar ist, und
wobei das Nahtmaterial (202) durch die Öffnung (210) hindurch, um die Kugel (206) herum und zurück nach draußen durch die Öffnung (210) gefädelt ist.

9. Das Knochenankersystem gemäß Anspruch 8, einen Stab (232) aufweisend, welcher eine Größe hat, um sich durch die Öffnung (210) am proximalen Ende (212) des geschlitzten Gehäuses (214) hindurch zu erstrecken, um die Kugel (206) innerhalb des Schlitzes (208) zu verlagern.

10. Das Knochenankersystem gemäß irgendeinem der Ansprüche 8 oder 9, wobei eine Zugspannung im Nahtmaterial (202) die Kugel (206) zu einer Oberseite (230) des Schlitzes (208) bewegt und das Nahtmaterial zwischen der Kugel und der Oberseite des Schlitzes zusammendrückt.

11. Das Knochenankersystem gemäß irgendeinem der Ansprüche 1 bis 7, wobei die Verriegelungsbaugruppe (150) ein Gehäuse (160) aufweist, welches mit dem Schlitz (162) bereitgestellt ist und sich am proximalen Ende (158) des Körperabschnitts (154) befindet,
wobei die Kugel (164) innerhalb des geschlitzten Gehäuses (160) verschiebbar ist, und
wobei das Nahtmaterial (152) oberhalb der Kugel (164) in den Schlitz (162) hinein, zum die Kugel herum und aus dem gleichen Schlitz unterhalb der Kugel zurück nach draußen gefädelt ist.

12. Das Knochenankersystem gemäß Anspruch 11, wobei eine Zugkraft an einem ersten Ende (174) des Nahtmaterials (152) die Kugel (164) zu einer Oberseite (182) des Schlitzes (162) bewegt, was eine Druckkraft erzeugt, welche das Nahtmaterial zwischen der Kugel und der Oberseite des Schlitzes zusammendrückt.

13. Das Knochenankersystem gemäß irgendeinem der vorhergehenden Ansprüche, einen Einsetzer (50; 90; 250) aufweisend, welcher am proximalen Ende (158) des Körperabschnitts (154; 204) angebracht ist.

14. Das Knochenankersystem gemäß Anspruch 13, wobei der Einsetzer (50; 90; 250) eine Bewegung der Eingriffselemente (156) von der Gefaltet-Konfiguration zur Entfaltet-Konfiguration verhindert.

15. Das Knochenankersystem gemäß irgendeinem der Ansprüche 13 oder 14, wobei der Einsetzer (50; 90; 250) einen Heizmechanismus (54) aufweist, um die Temperatur der Eingriffselemente (156) auf die zweite Temperatur zu erhöhen.

## Revendications

1. Système d'ancrage osseux pour coupler le tissu à l'os, le système d'ancrage osseux comprenant :
une partie de corps (154 ; 204) comprenant une extrémité proximale (158) et une extrémité distale adaptée pour être insérée dans l'os ;
une pointe sur l'extrémité distale de la partie de corps ;
un ensemble de verrouillage automatique (150 ; 200) positionné au niveau de l'extrémité proximale de la partie de corps ;
un matériau de suture (152; 202) mis en prise avec l'ensemble de verrouillage automatique ; et
un pluralité d'éléments de mise en prise en alliage à mémoire de forme activés par la température (156) fixés sur la partie de corps et maintenus dans une configuration rétractée à une première température, et déplaçables jusqu'à une configuration expansée à une seconde température supérieure à la première température,
**caractérisé en ce que** la pointe est adaptée pour pénétrer dans l'os sans trou pré-percé et **en ce que** l'ensemble de verrouillage (150 ; 200) comprend une bille (164 ; 206) coulissant le long d'un axe longitudinal (165) de la partie de corps dans une fente (162 ; 208) positionnée au niveau de l'extrémité proximale (158) de la partie de corps (154 ; 204).

2. Système d'ancrage osseux selon la revendication 1, dans lequel la première température est d'environ 25°C à environ 27°C et la seconde température est d'environ 30°c à environ 35°C.

3. Système d'ancrage osseux selon l'une quelconque des revendications précédentes, dans lequel le déplacement des éléments de mise en prise (156) de la configuration rétractée à la configuration expansée a lieu dans une plage de températures entre la première température et la seconde température.

4. Système d'ancrage osseux selon l'une quelconque des revendications précédentes, dans lequel la pointe comprend une section transversale supérieure à une section transversale des éléments de mise en prise (156) dans la configuration rétractée.

5. Système d'ancrage osseux selon l'une quelconque des revendications précédentes, dans lequel les éléments de mise en prise (156) subissent une expansion radialement vers l'extérieur à partir de la partie de corps (154; 204) lorsqu'ils sont dans la configuration expansée.

6. Système d'ancrage osseux selon l'une quelconque des revendications précédentes, dans lequel les éléments de mise en prise comprennent des structures à ardillons.

7. Système d'ancrage osseux selon l'une quelconque des revendications précédentes, dans lequel les éléments de mise en prise (156) sont configurés pour empêcher sensiblement le retrait de l'ancrage osseux de l'os.

8. Système d'ancrage osseux selon l'une quelconque des revendications précédentes, dans lequel l'ensemble de verrouillage (200) comprend :
un boîtier (214) muni de la fente (208) et positionné au niveau de l'extrémité proximale de la partie de corps (204) ; et
une ouverture (210) au niveau d'une extrémité proximale (212) du boîtier fendu (214) ;
dans lequel la bille (206) peut coulisser à l'intérieur du boîtier fendu (214) ; et
dans lequel le matériau de suture (202) est enfilé à travers l'ouverture (210), autour de la bille (206) et ressort par l'ouverture (210).

9. Système d'ancrage osseux selon la revendication 8, comprenant une tige (232) dimensionnée pour s'étendre à travers l'ouverture (210) au niveau de l'extrémité proximale (212) du boîtier fendu (214) pour déplacer la bille (206) à l'intérieur de la fente (208).

10. Système d'ancrage osseux selon l'une quelconque des revendications 8 ou 9, dans lequel la tension sur le matériau de suture (202) déplace la bille (206) jusqu'à un sommet (230) de la fente (208) et comprime le matériau de suture entre la bille et le sommet de la fente.

11. Système d'ancrage osseux selon l'une quelconque des revendications 1 à 7, dans lequel l'ensemble de verrouillage (150) comprend un boîtier (160) prévu avec la fente (162) et positionné au niveau de l'extrémité proximale (158) de la partie de corps (154) ;
dans lequel la bille (164) peut coulisser à l'intérieur du boîtier fendu (160) ; et
dans lequel le matériau de suture (152) est enfilé dans la fente (162) au-dessus de la bille (164), autour de la bille et ressort par la même fente au-dessous de la bille.

12. Système d'ancrage osseux selon la revendication 11, dans lequel une force de tension sur une première extrémité (174) du matériau de suture (152) fait avancer la bille (164) jusqu'à un sommet (182) de la fente (162), créant une force de compression qui comprime la suture entre la bille et le sommet de la fente.

13. Système d'ancrage osseux selon l'une quelconque des revendications précédentes, comprenant un dispositif d'insertion (50 ; 90 ; 250) fixé sur l'extrémité proximale (158) de la partie de corps (154 ; 204).

14. Système d'ancrage osseux selon la revendication 13, dans lequel le dispositif d'insertion (50 ; 90 ; 250) empêche le déplacement des éléments de mise en prise (156) de la configuration rétractée à la configuration expansée.

15. Système d'ancrage osseux selon l'une quelconque des revendications 13 ou 14, dans lequel le dispositif d'insertion (50 ; 90 ; 250) comprend un mécanisme de chauffage (54) pour augmenter la température des éléments de mise en prise (15fui) jusqu'à la seconde température.
